(19) Europäisches Patentamt
European Patent Office
Office européen des brevets

(11) Numéro de publication : **0 025 620**
**B1**

# (12) FASCICULE DE BREVET EUROPÉEN

(45) Date de publication du fascicule du brevet : **22.06.83**

(51) Int. Cl.³ : **C 07 C 50/16**, C 07 C 46/00

(21) Numéro de dépôt : **80200818.5**

(22) Date de dépôt : **01.09.80**

## (54) Procédé pour la fabrication d'anthraquinones substituées.

(30) Priorité : **13.09.79 FR 7923065**

(43) Date de publication de la demande : **25.03.81 Bulletin 81/12**

(45) Mention de la délivrance du brevet : **22.06.83 Bulletin 83/25**

(84) Etats contractants désignés : **AT BE CH DE FR GB IT LI LU NL SE**

(56) Documents cités : **FR A 2 154 476**

(73) Titulaire : **INTEROX Société anonyme dite:**
**Rue du Prince Albert, 33**
**B-1050 Bruxelles (BE)**

(72) Inventeur : **Schoemans, Jean**
**Boulevard Reyers, 167 - Boîte 24**
**B-1040 Bruxelles (BE)**
Inventeur : **Bouillet, Edmond**
**Avenue de l'Amarante, 6**
**B-1020 Bruxelles (BE)**

(74) Mandataire : **Meyers, Liliane et al**
**Solvay & Cie Département de la propriété industrielle**
**310, rue de Ransbeek**
**B-1120 Bruxelles (BE)**

EP 0 025 620 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue St-Denis, 75001 Paris, France

Procédé pour la fabrication d'anthraquinones substituées

La présente invention concerne un nouveau procédé pour la fabrication d'anthraquinones substituées qui sont utilisées comme intermédiaires de synthèse, notamment pour la fabrication de peroxyde d'hydrogène et la synthèse de colorants.

Il est connu de fabriquer des anthraquinones substituées par déshydratation des acides benzoylbenzoïques correspondants au moyen d'oléum (Brevet Etats-Unis 3 032 560 déposé le 29 mai 1959 au nom de L.H. DAWSEY). Ces procédés nécessitent la mise en œuvre de grandes quantités d'oléum, ce qui entraîne la formation d'acide sulfurique résiduaire pollué par des matières organiques, qu'il est très difficile de récupérer.

On a également essayé de préparer l'anthraquinone non substituée par réaction direction du chlorure de phtaloyle et de benzène (C. Friedel et J.M. Crafts, Ann. Chim. Phys., 1884, (6), *1*, p. 5 230). Le produit principal de cette réaction est cependant un phénylphtalide, l'anthraquinone n'étant produite qu'à l'état de traces.

Il est connu de fabriquer certains dérivés substitués de l'acridine ou de la xanthone par déshydratation des acides diphénylamine-2-carboxyliques et des éthers 2-carboxydiphényliques substitués correspondants au moyen d'acides tels que l'acide sulfurique ou l'acide polyphosphorique, à température élevée. Un tel procédé est décrit dans la demande de brevet FR-A-2 154 476 (The Wellcome Foundation Ltd). Il nécessite la mise en œuvre de grandes quantités de catalyseurs, ce qui entraîne la formation d'acides résiduaires pollués par des matières organiques qu'il est très difficile de récupérer.

La présente invention vise à fournir un procédé pour la fabrication d'anthraquinones substituées qui permet d'éviter la formation d'acide sulfurique résiduaire. Le procédé, qui est aisé à réaliser, permet d'obtenir de bons rendements et une bonne sélectivité car il ne donne pas lieu à la formation de quantités excessives de phénylphtalides.

L'invention concerne à cet effet un procédé pour la fabrication d'anthraquinones substituées répondant à la formule

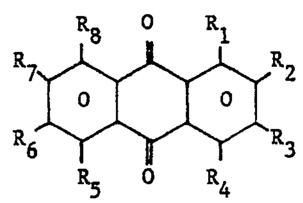

(I)

où les substituants $R_1$, $R_2$, $R_3$, $R_4$, $R_5$, $R_6$, $R_7$ et $R_8$ représentent un atome d'hydrogène ou un groupement carboné contenant de 1 à 12 atomes de carbone, $R_1$, $R_2$, $R_3$, $R_4$, $R_5$, $R_6$, $R_7$ et $R_8$ pouvant être identiques ou différents, un au moins parmi ces substituants représentant un groupement carboné et où les groupements carbonés sont des groupes alkyles, alkényles, aryles, alkylaryles, alkénylaryles, aralkyles, aralkényles, cycloalkyles ou cycloalkényles pouvant contenir dans leur structure au moins un hétéroatome constitué d'un halogène, d'oxygène, d'azote ou de soufre.

Les groupements carbonés précités peuvent éventuellement constituer deux à deux ($R_1$-$R_2$, $R_2$-$R_3$, $R_3$-$R_4$, $R_5$-$R_6$, $R_6$-$R_7$, et/ou $R_7$-$R_8$) une ou plusieurs chaînes carbonées uniques.

Selon l'invention, ces anthraquinones sont obtenues par cyclisation du chlorure d'acide correspondant de formule

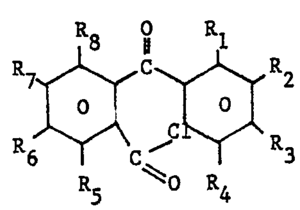

(II)

où les substituants ont la même signification que ci-dessus. Ces chlorures d'acides peuvent être fabriqués selon le procédé décrit dans une demande de brevet français n° 2 464 938, déposée par la Demanderesse le 13 septembre 1979.

Le procédé selon l'invention convient bien pour la fabrication d'anthraquinones substituées répondant à la formule I pour lesquelles les substituants $R_5$ et $R_7$ représentent tous deux un atome d'hydrogène. Il convient particulièrement bien pour la fabrication des anthraquinones substituées

répondant à la formule I dont les substituants $R_2$, $R_4$, $R_5$ et $R_7$ représentent tous un atome d'hydrogène.

Le procédé selon l'invention convient bien pour la fabrication d'anthraquinones substituées répondant à la formule I pour lesquelles le substituant $R_3$ représente un groupement carboné contenant au moins 2 atomes de carbone et de préférence un groupe alkyle contenant de 2 à 8 atomes de carbone. Les meilleurs résultats ont été obtenus dans le cas des anthraquinones dont les substituants $R_1$, $R_2$, $R_4$, $R_5$, $R_6$, $R_7$ et $R_8$ représentent l'hydrogène et le substituant $R_3$ représente un groupe alkyle contenant de 2 à 8 atomes de carbone. A titre d'exemples de telles anthraquinones substituées on peut citer la 2-éthylanthraquinone, les 2-propyl- et 2-isopropyl-anthraquinones, les 2-butyl-, 2-tert-butyl-, 2-isobutyl-anthraquinones et les 2-pentyl-, 2-sec-amyl- et 2-tert-amyl-anthraquinones.

La déshydrochloration peut se faire en présence ou en l'absence d'un catalyseur ajouté spécialement.

Comme catalyseur de la réaction, on utilise en général des catalyseurs du type Friedel-Crafts. Des exemples de ces catalyseurs sont donnés dans le livre Friedel-Crafts and Related Reactions de G. A. Olah, Volume I, 1963, Interscience Publ. On peut ainsi utiliser des acides de Brönsted ou des acides de Lewis. On utilise le plus souvent du chlorure d'hydrogène ou des acides de Lewis non protoniques. Parmi ceux-ci les halogénures et plus particulièrement les chlorures métalliques conviennent bien. De bons résultats ont été obtenus avec le chlorure d'aluminium. On peut également utiliser des mélanges de catalyseurs.

Ces catalyseurs sont en général mis en œuvre en quantités relativement importantes. Le plus souvent on utilise de 0,01 à 20 mol de catalyseur par mol de chlorure d'acide. De bons résultats ont été obtenus en mettant en œuvre de 1 à 5 mol de catalyseur par mol de chlorure d'acide.

La réaction est réalisée en général en phase liquide. Elle peut se faire en présence ou en l'absence d'un solvant. Les solvants mis en œuvre sont choisis parmi les solvants inertes dans les conditions de réaction. De préférence, on choisit des solvants capables de solubiliser le chlorure d'acide, ainsi que, le cas échéant, le catalyseur mis en œuvre. On peut utiliser un solvant unique ou un mélange de solvants.

Les solvants sont en général choisis parmi le dioxyde de soufre et les hydrocarbures substitués ou non. Ces hydrocarbures contiennent en général de 1 à 12 et de préférence de 1 à 8 atomes de carbone. Ils peuvent être de types divers et comporter des groupes tels que des groupes alkyles, alkényles, cycloalkyles, cycloalkényles, aryles, aralkyles, aralkényles, alkylaryles et alkénylaryles et des hétérocycles. Leurs substituants éventuels peuvent être de natures très diverses ; ils sont en général choisis parmi les atomes d'halogène ou de soufre et les groupes nitro, sulfone, sulfoxyde, éther et thioéther.

Les hydrocarbures halogénés et plus particulièrement les hydrocarbures chlorés, plus particulièrement ceux qui contiennent de 1 à 7 atomes de carbone, ainsi que le sulfure de carbone conviennent bien. De bons résultats ont été obtenus avec le sulfure de carbone, le tétrachlorométhane, le trichlorométhane, le dichlorométhane, le 1,2-dichloroéthane, le 1,2-dichloroéthylène, le trichloréthylène et le perchloroéthylène.

Les solvants éventuels peuvent être mis en œuvre en quantités variables. En général, le mélange réactionnel contient jusqu'à 95 % en poids de solvant.

La température de réaction peut varier dans de larges limites. Elle est en général comprise entre 300 et 600 K. La pression de réaction peut également varier dans de larges limites. La pression de réaction est en général choisie de manière à maintenir le mélange réactionnel sous forme liquide. La pression est le plus souvent comprise entre 0,1 et 100 bar.

Lorsque la déshydrochloration est effectuée en présence de catalyseur, on utilise en général un solvant tel que défini ci-avant. Dans ce cas, le mélange réactionnel contient habituellement de 30 à 95 % en poids de solvant. De bons résultats ont été obtenus en utilisant des mélanges réactionnels contenant de 50 à 90 % en poids de solvant. En présence de catalyseurs, la température est choisie en général inférieure à 400 K. Le plus souvent, elle est comprise entre 300 et 370 K. La pression est alors en général comprise entre 0,1 et 5 bar.

Lorsque la déshydrochloration est effectuée en l'absence substantielle d'un catalyseur extérieur (soit en présence de moins de 0,1 mol de catalyseur tel que défini ci-avant, par mol de chlorure d'acide), on utilise en général des températures de réaction supérieures à 350 K. Le plus souvent on utilise des températures comprises entre 370 et 600 K. De bons résultats ont été obtenus en utilisant des températures comprises entre 400 et 550 K. La pression est dans ce cas en général comprise entre 0,8 et 100 bar et le plus souvent entre 1 et 80 bar.

Lorsqu'on opère en l'absence de catalyseur extérieur, il est vraisemblable que la réaction est catalysée par le chlorure d'hydrogène produit par la réaction.

Comme indiqué ci-dessus, la réaction en présence ou en l'absence de catalyseur extérieur est réalisée en général en milieu liquide. Ce milieu peut éventuellement être maintenu à l'ébullition. La réaction peut être réalisée dans des réacteurs soumis à un vide partiel ou dans des réacteurs capables de fonctionner sous pression élevée selon les pressions de réaction. Les réacteurs utilisés sont en général réalisés dans des matériaux susceptibles de résister à la corrosion.

La réaction peut se faire en continu ou en discontinu dans un réacteur unique ou à étages.

Afin d'illustrer l'invention, sans pour autant en limiter la portée, on donne ci-après des exemples de fabrication d'anthraquinones substituées. Les exemples 1, 2, 3, 4, 6, 7, 8, 9, 10 et 11 ont été réalisés en présence d'un catalyseur, les exemples 5 et 12 ont été réalisés en l'absence de catalyseur ajouté spécialement.

Fabrication de 2-éthyl-anthraquinone

Exemple 1

On prend une solution à 20 % en poids de chlorure d'acide ortho-(4'-éthylbenzoyl)-benzoïque (pureté 94 %) dans du 1,2-dichloréthane. On maintient la solution à 288 K et on introduit en 1 h 20 min, 3 mol de chlorure d'aluminium par mol de chlorure d'acide.

On porte le mélange à 303 K et on le maintient pendant 4 h à cette température.

On obtient la 2-éthylanthraquinone avec un rendement de 39,9 % et une sélectivité de 81 %.

Fabrication d'un mélange de 2-sec- et 2-tert-amyl-anthraquinones
(rapport des deux isomères d'environ 1 : 1)

Exemple 2

On prend une solution à 20 % en poids d'un mélange de chlorures d'acides ortho-(4'-sec- et 4'-tert-amylbenzoyl)-benzoïques (pureté 92 %) dans le 1,2-dichloréthane. On introduit dans la solution en 1 h 20 min, 3 mol de chlorure d'aluminium par mol de chlorure d'acide.

On porte le mélange à 323 K et on le maintient pendant 5 h à cette température.

On obtient le mélange de 2-sec- et 2-tert-amylanthraquinones avec un rendement de 87,3 % et une sélectivité de 94 %.

Exemple 3

On prend une solution à 20 % en poids d'un mélange de chlorures d'acides ortho-(4'-sec-et 4'-tert-amylbenzoyl)-benzoïques dans le sulfure de carbone et on procède comme à l'exemple 2.

Après 3 h 30 min de réaction à 313 K, on obtient le mélange de 2-sec- et 2-tert-amylanthraquinones avec un rendement de 79 % et une sélectivité de 84 %.

Exemple 4

On prend une solution à 20 % en poids d'un mélange de chlorures d'acides ortho-(4'-sec- et 4'-tert-amylbenzoyl)-benzoïques dans le tétrachlorométhane et on procède comme à l'exemple 2. Après 3 h 30 min de réaction à 323 K, on obtient le mélange de 2-sec- et 2-tert-amylanthraquinones avec un rendement de 70 % et une sélectivité de 84 %.

Exemple 5

On dispose 50 g d'un mélange de chlorures d'acide ortho-(4'-sec- et 4'-tert-amylbenzoyl)-benzoïques dans un autoclave en acier inoxydable maintenu sous pression de 20 bar sous atmosphère d'azote. On maintient la température à 498 K.

On obtient le mélange de 2-sec- et 2-tert-amylanthraquinones avec un rendement de 55,3 % et une sélectivité de 80 %.

Fabrication de 2-tert-butyl-anthraquinone

Exemple 6

On prend une solution à 20 % en poids de chlorure d'acide ortho-(4'-tert-butylbenzoyl)-benzoïque dans le 1,2-dichloréthane. On introduit dans la solution en 1 h 20 min, 3,09 mol de chlorure d'aluminium par mol de chlorure d'acide.

On porte le mélange à 313 K et on le maintient pendant 5 h à cette température.

On obtient la 2-tert-butyl-anthraquinone avec un rendement de 91,6 % et une sélectivité de 97 %.

Exemple 7

On prend une solution à 20 % en poids de chlorure d'acide ortho-(4'-tert-butylbenzoyl)-benzoïque dans le dichlorométhane. On introduit dans la solution en 1 h 20 min, 3,09 mol de chlorure d'aluminium par mol de chlorure d'acide.

On porte le mélange à 308 K et on le maintient pendant 6 h à cette température.

On obtient la 2-tert-butyl-anthraquinone avec un rendement de 79,1 % et une sélectivité d'environ 100 %.

Exemple 8

On procède comme à l'exemple 7 en utilisant du 1,2-dichloréthylène comme solvant et une

température de réaction de 313 K.

On obtient la 2-tert-butyl-anthraquinone avec un rendement de 79,1 % et une sélectivité d'environ 100 %.

Exemple 9

On procède comme à l'exemple 7 en utilisant du perchloréthylène comme solvant et une température de 323 K.

On obtient la 2-tert-butyl-anthraquinone avec un rendement de 81,5 % et une sélectivité de 85 %.

Exemple 10

On procède comme à l'exemple 7 en utilisant du tétrachlorure de carbone comme solvant et une température de 323 K.

On obtient la 2-tert-butyl-anthraquinone avec un rendement de 90,3 % et une sélectivité de 97 %.

Exemple 11

On procède comme à l'exemple 7 en utilisant du sulfure de carbone comme solvant et une température de 313 K.

On obtient la 2-tert-butyl-anthraquinone avec un rendement de 93,3 % et une sélectivité d'environ 100 %.

Exemple 12

On dispose 90 g de chlorure d'acide ortho-(4'-tert-butylbenzoyl)-benzoïque dans un autoclave en acier inoxydable maintenu sous une pression de 20 bar sous atmosphère d'azote. On maintient la température à 473 K.

On obtient la 2-tert-butyl-anthraquinone avec un rendement de 85,6 % et une sélectivité du 97 %.

**Revendications**

1. Procédé pour la fabrication d'anthraquinones substituées répondant à la formule

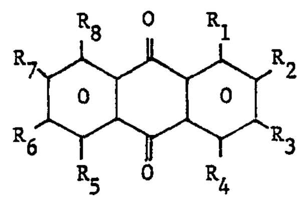

où les substituants $R_1$, $R_2$, $R_3$, $R_4$, $R_5$, $R_6$, $R_7$ et $R_8$ représentent un atome d'hydrogène ou un groupement carboné contenant de 1 à 12 atomes de carbone, $R_1$, $R_2$, $R_3$, $R_4$, $R_5$, $R_6$, $R_7$ et $R_8$ pouvant être identiques ou différents, un au moins parmi ces substituants représentant un groupement carboné et où les groupements carbonés sont des groupes alkyles, alkényles, aryles, alkylaryles, alkénylaryles, aralkyles, aralkényles, cycloalkyles ou cycloalkényles pouvant contenir dans leur structure au moins un hétéroatome constitué d'un halogène, d'oxygène, d'azote ou de soufre, caractérisé par la déshydrochloration des chlorures d'acides correspondants de formule

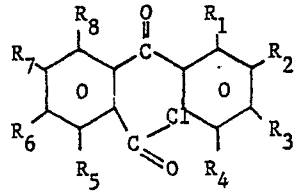

où $R_1$, $R_2$, $R_3$, $R_4$, $R_5$, $R_6$, $R_7$ et $R_8$ ont la même signification.

2. Procédé selon la revendication 1, caractérisé en ce que le groupement carboné $R_3$ contient au moins 2 atomes de carbone.

3. Procédé selon la revendication 1 ou 2, caractérisé en ce que $R_2$, $R_4$, $R_5$ et $R_7$ représentent un atome d'hydrogène.

4. Procédé selon la revendication 3, caractérisé en ce que $R_1$, $R_6$ et $R_8$ représentent également un atome d'hydrogène.

5. Procédé selon l'une quelconque des revendications 1 à 4, caractérisé en ce que $R_3$ représente un groupe alkyle contenant de 2 à 8 atomes de carbone.

6. Procédé selon l'une quelconque des revendications 1 à 5 caractérisé en ce qu'on met en œuvre un catalyseur du type Friedel-Crafts.

7. Procédé selon la revendication 6, caractérisé en ce qu'on met en œuvre de 1 à 5 mole de catalyseur par mole de chlorure d'acide.

8. Procédé selon la revendication 6 ou 7, caractérisé en ce qu'on met en œuvre du chlorure d'aluminium.

9. Procédé selon l'une quelconque des revendications 6 à 8, caractérisé en ce qu'on opère à une température comprise entre 300 et 370 K.

10. Procédé selon l'une quelconque des revendications 1 à 5, caractérisé en ce qu'on opère à une température comprise entre 400 et 550 K en l'absence de catalyseur ajouté spécialement.

11. Procédé selon l'une quelconque des revendications 1 à 10, caractérisé en ce qu'on met en œuvre un solvant.

12. Procédé selon la revendication 11, caractérisé en ce que le solvant est choisi parmi le sulfure de carbone et les hydrocarbures chlorés contenant de 1 à 7 atomes de carbone.

**Claims**

1. Process for the manufacture of substituted anthraquinones corresponding to the formula

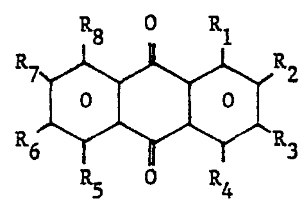

in which the substituents $R_1$, $R_2$, $R_3$, $R_4$, $R_5$, $R_6$, $R_7$ and $R_8$ represent a hydrogen atom or a hydrocarbon group containing from 1 to 12 carbon atoms, it being possible for $R_1$, $R_2$, $R_3$, $R_4$, $R_5$, $R_6$, $R_7$ and $R_8$ to be identical or different and at least one of these substituents representing a hydrocarbon group, and in which the hydrocarbon groups are alkyl, alkenyl, aryl, alkylaryl, alkenylaryl, aralkyl, aralkenyl, cycloalkyl or cycloalkenyl groups which can contain, in their structure, at least one hetero-atom consisting of a halogen, oxygen, nitrogen or sulphur, characterised by the dehydrochlorination of the corresponding acid chlorides of the formula

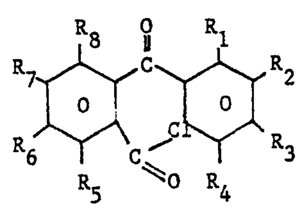

in which $R_1$, $R_2$, $R_3$, $R_4$, $R_6$, $R_6$, $R_7$ and $R_8$ have the same meaning.

2. Process according to Claim 1, characterised in that the hydrocarbon group $R_3$ contains at least 2 carbon atoms.

3. Process according to Claim 1 or 2, characterised in that $R_2$, $R_4$, $R_5$ and $R_7$ represent a hydrogen atom.

4. Process according to Claim 3, characterised in that $R_1$, $R_6$ and $R_8$ also represent a hydrogen atom.

5. Process according to any one of Claims 1 to 4, characterised in that $R_3$ represents an alkyl group containing from 2 to 8 carbon atoms.

6. Process according to any one of Claims 1 to 5, characterised in that a catalyst of the Friedel-Crafts type is used.

7. Process according to Claim 6, characterised in that from 1 to 5 mols of catalyst are used per mol of acid chloride.

8. Process according to Claim 6 or 7, characterised in that aluminium chloride is used.

9. Process according to any one of Claims 6 to 8, characterised in that the reaction is carried out at a temperature of between 300 and 370 K.

10. Process according to any one of Claims 1 to 5, characterised in that the reaction is carried out at a temperature of between 400 and 550 K in the absence of specially added catalyst.

11. Process according to any one of Claims 1 to 10, characterised in that a solvent is used.

12. Process according to Claim 11, characterised in that the solvent is chosen from amongst carbon disulphide and chlorohydrocarbons containing from 1 to 7 carbon atoms.

**Ansprüche**

1. Verfahren zur Herstellung substituierter Anthrachinone entsprechend der Formel

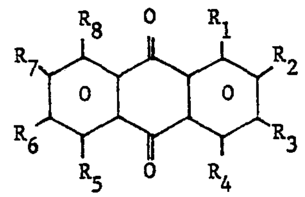

worin die Substituenten $R_1$, $R_2$, $R_3$, $R_4$, $R_5$, $R_6$, $R_7$ und $R_8$ ein Wasserstoffatom oder eine 1 bis 12 Kohlenstoffatome aufweisende kohlenstoffhaltige Gruppe bedeuten, wobei $R_1$, $R_2$, $R_3$, $R_4$, $R_5$, $R_6$, $R_7$ und $R_8$ gleich oder voneinander verschieden sein können und wobei wenigstens einer dieser Substituenten eine kohlenstoffhaltige Gruppe darstellt und worin die kohlenstoffhaltigen Gruppen Alkylgruppen, Alkenylgruppen, Arylgruppen, Alkylarylgruppen, Alkenylarylgruppen, Aralkylgruppen, Aralkenylgruppen, Cycloalkylgruppen oder Cycloalkenylgruppen sind, die in ihrer Struktur wenigstens ein Heteroatom, bestehend aus einem Halogen, Sauerstoff, Stickstoff oder Schwefel, enthalten können, gekennzeichnet durch die Dehydrochlorierung der entsprechenden Säurechloride der Formel

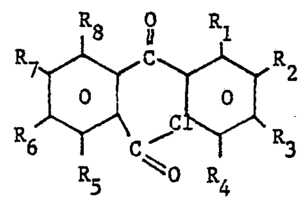

worin $R_1$, $R_2$, $R_3$, $R_4$, $R_5$, $R_6$, $R_7$ und $R_8$ die gleiche Bedeutung haben.

2. Verfahren nach Anspruch 1, dadurch gekennzeichnet, dass die kohlenstoffhaltige Gruppe $R_3$ wenigstens 2 Kohlenstoffatome enthält.

3. Verfahren nach Anspruch 1 oder 2, dadurch gekennzeichnet, dass $R_2$, $R_4$, $R_5$ und $R_7$ ein Wasserstoffatom darstellen.

4. Verfahren nach Anspruch 3, dadurch gekennzeichnet, dass $R_1$, $R_6$ und $R_8$ gleichfalls ein Wasserstoffatom darstellen.

5. Verfahren nach einem der Ansprüche 1 bis 4, dadurch gekennzeichnet, dass $R_3$ eine 2 bis 8 Kohlenstoffatome aufweisende Alkylgruppe darstellt.

6. Verfahren nach einem der Ansprüche 1 bis 5, dadurch gekennzeichnet, dass man einen Katalysator vom Friedel-Crafts-Typus einsetzt.

7. Verfahren nach Anspruch 6, dadurch gekennzeichnet, dass man 1 bis 5 Mole Katalysator je Mol Säurechlorid einsetzt.

8. Verfahren nach Anspruch 6 oder 7, dadurch gekennzeichnet, dass man Aluminiumchlorid einsetzt.

9. Verfahren nach einem der Ansprüche 6 bis 8, dadurch gekennzeichnet, dass man bei einer Temperatur zwischen 300 und 370 K arbeitet.

10. Verfahren nach einem der Ansprüche 1 bis 5, dadurch gekennzeichnet, dass man bei einer Temperatur zwischen 400 und 550 K und in Abwesenheit eines speziell zugesetzten Katalysators arbeitet.

11. Verfahren nach einem der Ansprüche 1 bis 10, dadurch gekennzeichnet, dass man ein Lösungsmittel anwendet.

12. Verfahren nach Anspruch 11, dadurch gekennzeichnet, dass das Lösungsmittel ausgewählt wird unter Schwefelkohlenstoff und den chlorierten Kohlenwasserstoffen mit 1 bis 7 Kohlenstoffatomen.